# EUROPEAN PATENT SPECIFICATION

(11) **EP 3 867 282 B1**
(45) Date of publication and mention of the grant of the patent: **23.11.2022**
(21) Application number: 19798724.1
(22) Date of filing: 11.10.2019
(51) Int. Cl.: C08B 37/08, A61K 31/728, C08L 5/08, A61K 47/54, A61K 47/55, A61K 47/61

(54) **CONJUGATES OF HYALURONIC ACID AND AMINOBISPHOSPHONATES AND THE THERAPEUTIC USE THEREOF**
KONJUGATE VON HYALURONSÄURE UND AMINOBISPHOSPHONATEN UND IHRE THERAPEUTISCHE VERWENDUNG
CONJUGUÉS D'ACIDE HYALURONIQUE ET D'AMINOBISPHOSPHONATES ET LEUR UTILISATION THÉRAPEUTIQUE

(30) Priority: 15.10.2018 IT 201800009444
(43) Date of publication of application: 25.08.2021
(73) Proprietor: Fidia Farmaceutici S.p.A., 35031 Abano Terme (PD) (IT)
(72) Inventor: PLUDA, Stefano, 35031 Abano Terme (PD) (IT); PAVAN, Mauro, 35031 Abano Terme (PD) (IT); BARBERA, Carlo, 35031 Abano Terme (PD) (IT)
(74) Representative: Bianchetti & Minoja SRL
(86) International application number: PCT/IB2019/058698
(87) International publication number: WO 2020/079551

(56) References cited:
- WO-A1-01/66601
- SUJIT KOOTALA ET AL: "Bisphosphonate-functionalized hyaluronic acid showing selective affinity for osteoclasts as a potential treatment for osteoporosis", BIOMATERIALS SCIENCE, vol. 3, no. 8, 1 August 2015 (2015-08-01), pages 1197-1207, XP055600793, GB ISSN: 2047-4830, DOI: 10.1039/C5BM00096C
- NEJADNIK M REZA ET AL: "Self-healing hybrid nanocomposites consisting of bisphosphonated hyaluronan and calcium phosphate nanoparticles", BIOMATERIALS, ELSEVIER SCIENCE PUBLISHERS BV., BARKING, GB, vol. 35, no. 25, 24 May 2014 (2014-05-24), pages 6918-6929, XP028868100, ISSN: 0142-9612, DOI: 10.1016/J.BIOMATERIALS.2014.05.003

## Description

### Field of invention

The invention relates to conjugates of hyaluronic acid (HA) and amino-bisphosphonates (N-BP) for use in the intra-articular and/or locoregional treatment of osteoarthrosis, and its repercussions at cartilage and subchondral level.

### State of the art

Osteoarthrosis is a complex, multifactorial disorder involving the cartilage and subchondral bone, wherein changes in the subchondral bone have repercussions on the cartilage due to "crosstalk". It can involve all the joints of the body, but the areas most affected in general are the hands (fingers and base of the thumb), neck, knees and hips. The most common symptoms include pain, stiffness, reduced motor capacity, swelling and joint effusions. One of the most typical characteristics of osteoarthrosis is impoverishment of synovial fluid in terms of both the concentration and the average molecular weight of hyaluronic acid (HA), which depolymerises, losing its viscoelastic properties, and consequently altering the rheology of the synovial fluid (Balazs EA. et al., J Rheumatol Suppl, 1993, 12:75-82; Belcher C. et al., Annals Rheum Dis, 1997, 56:299-307). The HA contained in the synovial fluid acts as a viscous lubricant during slow movements, while its elastic properties enable it to absorb traumas or microtraumas affecting the joint during rapid movements. Its depolymerisation therefore significantly alters the functioning of the joints (Balazs EA, 1974, in "Disorders of the Knee", Lippincott Co, Philadelphia, p. 63-75).

In general, HA is a linear-chain heteropolysaccharide consisting of alternating residues of D-glucuronic acid and N-acetyl-D-glucosamine, with a molecular weight (MW) ranging from 400 to 13 × 10⁶ Da, depending on the source from which it is obtained and the preparation methods used. It is present in nature in pericellular gels, in the ground substance of the connective tissue of vertebrates, in the vitreous humour and the umbilical cord and, as stated, in the synovial fluid of the joints.

HA plays an important role in biological organisms, especially as a mechanical support for the cells of many tissues, such as skin, tendons, muscles and cartilage.

Through its CD44 membrane receptor, it modulates many cell processes such as cell proliferation, migration and differentiation, and angiogenesis, and also performs other functions, such as tissue hydration and joint lubrication. The protective effect of HA against the cartilage degeneration that can be caused when a joint is damaged by disease or trauma is also well known; in said situation, a strong concentration of pro-inflammatory cytokines, especially interleukin-1 (IL-1), is present in the joint cavity, promoting cartilage disintegration and inhibiting chondrocyte proliferation (van Beuningen H.M. et al., Arthritis Rheum, 1991, 34:606-615). Various scientific experiments demonstrate that HA counteracts the action of IL-1, drastically reducing its adverse effects and repairing the cartilage tissue of the joint into which it is injected (Stove J. et al., J Orthop Res, 2002, 20:551-555).

Finally, it has been demonstrated that HA has a painkilling effect, acting as partial agonist of the k-opioid receptors (Zavan B. et al., *PLoS One*, 2013; 8, e55510).

In view of the combination of said characteristics, it is one of the products most widely used in the treatment of osteoarticular and cartilage disorders, in the form of intra-articular infiltrations based on HA "as is" or in its derivatised forms, using chemical methods (crosslinking) or physical methods (irradiation, etc.).

In addition to HA and many other well-known medicaments, bisphosphonates (BP), a class of medicaments which have been known for some time, are administered for the treatment of osteoarthrosis in clinical practice. They inhibit bone resorption by the osteoclasts, which justifies their widespread use in all disorders requiring bone consolidation. BPs are also used to treat osteoporosis, Paget's disease, bone metastases (in the presence or absence of hypercalcaemia), multiple myeloma, and all the other conditions that can cause bone fragility; for example, they play an important part in preventing osteoporosis induced by chronic use of corticosteroids. BPs also act as anti-inflammatories, due to their inhibitory effect on the matrix metalloproteases, especially MMP-13 (Heikkila et al., Anticancer Drugs, 2002, 13, 245-254); MMP-13s are known to be involved in the inflammatory processes characteristic of osteoarticular diseases (such as osteoarthrosis and rheumatoid arthritis), which cause serious damage to the cartilage and bone tissue of the joint, and to the synovial fluid and the tendons.

BPs are divided into two main groups: non-nitrogen-containing or first-generation bisphosphonates (etidronate, clodronate and tiludronate) and nitrogen-containing or second-generation bisphosphonates. In particular, the latter include amino-bisphosphonates (pamidronate, alendronate, ibandronate and neridronate) which contain a nitrogen atom as part of an amino group, and are used in the present invention. Finally, the third-generation BP zolendronate contains two nitrogen atoms in an imidazole ring.

BPs are usually administered by injection (intravenous or intramuscular) or orally.

The oral bioavailability of the second-generation BPs is extremely low, with values around or below 1%, and even lower when they are taken with a meal. Moreover, about 78% thereof bonds effectively with the plasma proteins, while half the remainder is excreted in the urine within 24 hours, and only the other half is redistributed in the bone, aspecifically with respect to target site. This means that in order to obtain a pharmacological effect, fairly high doses of the active ingredient must be administered to ensure that a pharmacologically active amount reaches the site of action.

Whichever administration route is used, the side effects are fairly severe, ranging from headache and dizziness, rash, nausea and diarrhoea to oesophageal ulcers, after oral administration. In the case of parenteral administration, the most common, significant side effects are fever myalgia and skeletal pain. Chronic administration can even cause osteonecrosis of the lower jaw.

It is therefore important to have access to a product that exploits the pharmacological properties of the BPs, but has low toxicity and can be conveyed directly to the desired site, thus avoiding dispersion in the body.

EP 1994945 discloses the formation of HA-alendronate (HA-ALD) conjugates obtained by direct amido bond between the ALD amine and the HA carboxyl. The resulting conjugate is not hydrolysable: it is well known that the amido bond is a strong, stable, non-hydrolysable bond, especially under physiological conditions.

Nejadnik et al. (Biomaterials, 2014, 35, 6918-29) describe similar derivatives with a molecular spacer; these are hydrazide derivatives of HA with sulphydryl functionality able to bind to acrylic derivatives of ALD by UV irradiation and the use of a photoinitiator. Once again, the bond between HA and ALD is not hydrolysable under physiological conditions.

EP 1284754 describes a physical mixture between a BP and an agent able to prevent its immediate diffusion (including HA), after subcutaneous injection. The authors affirm that gradual release allows BP concentrations exceeding those normally used to be employed when they are in a simple aqueous solution. As will be clear from the following description of the present invention, EP 1284754 not only describes a product different from the conjugates according to the invention, as it relates to a simple physical mixture, but also pursues an entirely different purpose, because one of the aims of the invention is to use lower doses of BP than the standard doses, so as to obtain a product wherein HA and BP produce synergic effects.

### Description of the invention

The invention relates to a conjugate between hyaluronic acid (HA) and an amino-bisphosphonate (N-BP) wherein conjugation takes place with the use of a spacer **L** consisting of a linear alkyl chain or a polyoxyethylene chain. In the conjugate according to the invention, the HA carboxyl is bonded via an ester bond to spacer **L**, which in turn is bonded to the nitrogen of the N-BP via a carbamic bond.

The conjugate according to the invention has the general formula (I) 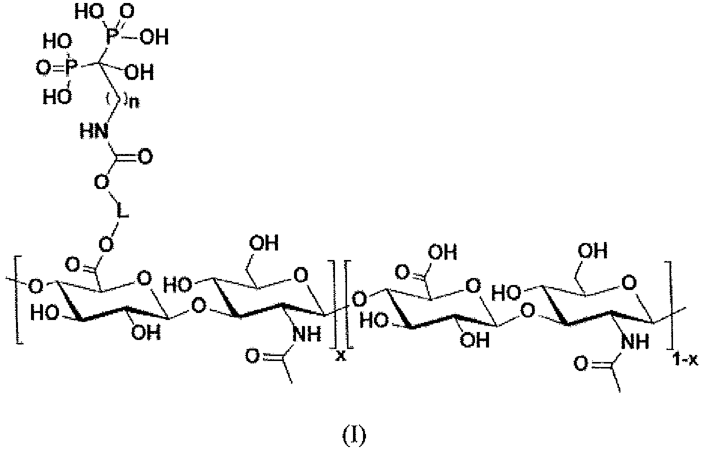 wherein:
- **L** is a spacer of formula -(CH₂)ₘ-, wherein **m** is an integer from 2 to 10; or **L** is a spacer of formula -(CH₂CH₂O)ₚ-CH₂CH₂- wherein **p** is an integer from 1 to 4;
- **n** is an integer from 2 to 5;
- **x** is a number that represents the degree of substitution (DS) of HA with N-BP in relation to the HA carboxyls, and ranges from 0.05 to 0.30 moles of N-BP/mole of HA;
- the weight-average molecular weight of HA is from 30000 Da to 3×10⁶ Da.

The invention also includes the salts of the conjugate of formula (I), wherein the phosphonic groups of the amino-bisphosphonic moiety and the HA carboxyl groups not involved in the conjugation with N-BP are partly or completely salified with an alkali or alkaline-earth metal cation, the ammonium cation or a (C₁-C₄)tetraalkylammonium cation, preferably an alkali metal cation, and more preferably the Na⁺ cation.

Following enzymatic hydrolysis or hydrolysis at acid or basic pHs or under physiological conditions, the ester bond and the carbamic bond of the conjugate of formula (I) cleave, and the active ingredient (N-BP) is released *in situ* together with HA, which can thus exert its well-known rheological, biocompatibility and cell recognition characteristics. Following hydrolysis, spacer **L** gives rise to an α,ω-alkyl diol or a low-molecular-weight polyethylene glycol which, at the concentrations used and administered by the intra-articular and/or locoregional route, are practically devoid of toxicity.

The invention also relates to pharmaceutical compositions containing a conjugate of formula (I) and at least one pharmaceutically acceptable excipient and/or carrier, and the therapeutic uses of the conjugates of formula (I) and the formulations thereof, in particular for visco supplementation.

A further object of the invention is a viscosupplement comprising a conjugate of formula (I) and at least one pharmaceutically acceptable excipient and/or carrier.

The conjugates according to the invention, the pharmaceutical compositions thereof and the viscosupplements that contain them are intended for use in the intra-articular and/or locoregional treatment of osteoarthrosis and its repercussions at cartilage and subchondral level; in the treatment of post-menopausal or drug-induced osteoporosis; in the treatment of bone fragility due to trauma or disease; and in the intraosseous and/or locoregional treatment of disorders characterised by altered metabolic bone turnover. They are also useful in promoting osseointegration of prostheses, i.e. when a close connection needs to be created between a prosthesis (generally, but not necessarily, made of titanium) and the bone tissue into which it is inserted.

### Description of figures

Figure 1. Quantitation of *in vitro* release of alendronate (ALD) from HA-ALD conjugate.
Figure 2. Comparison of the values of the viscoelastic moduli of the HA-ALD conjugate of Example 3, HA, and a physical mixture of HA and ALD.
Figure 3. Comparison between the dynamic viscosity η of the HA-ALD conjugate of Example 3, a physical mixture of HA and ALD, HA, Hymovis^{®} and Synvisc^{®}.
Figure 4. Cytotoxicity of 25 µM, 50 µM and 100 µM concentrations of ALD and of the HA-ALD conjugate of Example 3 on Saos-2 osteoblasts after 3 (Figure 4A) and 7 (Figure 4B) days of incubation.
Figure 5. Cytotoxicity of 25 µM, 50 µM and 100 µM concentrations of ALD and of the HA-ALD conjugate of Example 3 towards primary bovine chondrocytes after 3 (Figure 5A) and 7 (Figure 5B) days of incubation.
Figure 6. Quantitation of soluble collagen released following inflammatory stimulus by a sample of cartilage harvested from an adult bovine femur and treated with the HA-ALD conjugate of Example 3.

### Detailed description of the invention

The number **x** represents the degree of substitution (DS) of HA with N-BP, i.e. the fraction of the carboxyl groups of the HA repeating unit which are involved in forming the conjugate with N-BP. In the conjugates according to the invention, **x** is a number from 0.05 to 0.30; in other words the HA carboxyl groups involved in the conjugation range from 5% to 30% on a molar basis of the total carboxyl groups available for conjugation.

The degree of substitution **x** of HA with N-BP in relation to the HA carboxyls preferably ranges from 0.10-0.30 mole/mole (DS from 10% to 30% mol/mol), and more preferably from 0.10 to 0.20 mole/mole (DS from 10% to 20% mol/mol).

The HA used according to the invention can derive from any source, such as rooster combs (EP138572), fermentation (from *Streptococcus equi* or *zooepidemicus,* EP0716688) or biosynthesis (from *Bacillus,* EP2614088, EP2614087), and can be purified by various techniques (WO2018020458, IT102017000081449).

The weight-average molecular weight of HA ranges from 30000 Da to 3×10⁶ Da, in particular from 1×10⁵ Da to 1×10⁶ Da, more preferably from 150000 Da to 800000 Da, and even more preferably from 170000 Da to 230000 Da or from 500000 Da to 730000 Da. "Weight-average molecular weight" here means that calculated by the "intrinsic viscosity" method (Terbojevich et al., Carbohydr Res, 1986, 363-377).

The N-BP is preferably selected from pamidronate, neridronate and alendronate. Alendronate (ALD) is particularly preferred.

When spacer **L** is a straight aliphatic chain of formula -(CH₂)ₘ-, the number of carbon atoms **m** preferably ranges from 2 to 5, and even more preferably is 2.

When spacer **L** is a spacer of formula -(CH₂CH₂O)ₚ-CH₂CH₂-, **p** is preferably 1.

The conjugate of formula (I), wherein L is a straight aliphatic chain of formula -(CH₂)ₘ- wherein **m** is 2, is particularly preferred.

The conjugates of formula I can be prepared by reacting a derivative of N-BP, preferably of a tetrabutylammonium (TBA) salt, with a compound of formula X-L-OA, wherein **L** is as defined above, **X** is a leaving group, typically a halogen atom, and A is a hydroxy activating group. The resulting derivative is then reacted, even without being isolated, with a hyaluronic acid derivative, typically a tetrabutylammonium salt in a solvent such as dimethylsulphoxide or dimethylformamide. In the case of conjugates wherein **L** is a spacer of formula -(CH₂)ₘ- wherein **m** is 2, the preferred compound of formula X-L-OA is 2-chloroethyl-1H-imidazole-1-carboxylate. The conjugates are typically isolated in the form of disodium salts at the moiety of N-BP and monosodium salts at the HA carboxyl by adding a saturated solution of sodium chloride followed by dialysis at pH 6 and final freeze-drying.

The conjugation of HA as described represents a macromolecular drug delivery system for N-BP; after administration, as the hydrolysis progresses, the conjugate releases the active ingredient, N-BP, which acts on bone resorption, and HA, which returns to its native form and performs the well-known actions of lubrication, viscosupplementation, painkilling effect by acting on the nociceptors, etc.

The conjugate in solution can be sterilised by filtration or by heat treatment in an autoclave, can be formulated in aqueous solutions (such as water, saline solution or PBS), at a pH between 6 and 7 (i.e. a physiological pH), and can be extruded even with small-gauge needles.

The conjugate of the invention also has highly unusual, unexpected rheological characteristics; at the time of its preparation and use it takes the form of a viscous solution, but in the presence of bivalent ions, in particular calcium ions, and depending on the concentration and degree of substitution, its viscosity changes radically and a compact gel is created, with viscoelastic characteristics comparable or even superior to those of the most widely used chemically crosslinked viscoelastics. This unusual characteristic is extremely important, because calcium ions are normally present in synovial fluid (Madea et al., Forensic Sci Int, 2001, 118, 29-35), even when it is "impoverished" by osteoarthrosis. This means that the conjugate of the invention can be administered intra-articularly in the form of a viscous solution, with the undeniable advantages that this involves, such as ease of extrusion, and is then converted to a compact gel, without the addition of other substances (such as crosslinking agents) or physical treatments (UV radiation, etc.).

The conjugate of the invention can be used:
- in the intra-articular and/or locoregional treatment of osteoarthrosis, post-menopausal osteoporosis or osteoporosis induced by drugs such as steroids, bone fragility due to trauma or diseases such as multiple myeloma and bone metastasis;
- in the intraosseous and/or locoregional treatment of all disorders characterised by altered metabolic bone turnover, such as Paget's disease;
- to improve the osseointegration of prostheses.

For therapeutic purposes, the conjugate of the invention is used in the form of a pharmaceutical composition containing a therapeutically effective amount of said conjugate and at least one pharmaceutically acceptable excipient and/or carrier. Said formulations can be prepared by conventional methods, such as those described in Remington's Pharmaceutical Sciences, 18th ed., Mack Publishing Co. The doses of the conjugates according to the invention will obviously depend on the administration route and other parameters such as the patient's weight and age and the severity of the disorder, and can also be determined by the doctor on the basis of known information about the effective clinical doses of bisphosphonates.

The conjugate according to the invention, in addition to its drug delivery system (DDS) properties for the release of N-BP and HA, also offers greater efficacy and lower toxicity than similar doses of N-BP administered directly.

The rheological characteristics acquired by the conjugate according to the invention in the presence of bivalent ions also mean that it is an excellent candidate for 3D printing and/or bioprinting applications, such as the creation of parts of bone structures missing as a result of traumas, orthopaedic surgery, etc.

A further application of the conjugate of the invention is treatment and filling of subchondral bone lesions, including bone marrow lesions, by radioguided intraosseous infusion of the conjugate claimed herein. Due to its rheological properties it mechanically consolidates the subchondral lesion, acting as a kind of bone cement, and above all exploits the chondroprotective and osteogenic properties of N-BP, thus actively contributing to the repair of the lesion.

The conjugate of the invention innovatively solves the technical problem of providing a product that exploits the pharmacological properties of BPs, but has low toxicity and can be conveyed directly to the desired site so as to avoid dispersions in the body, in that:
1. it is a controlled-release N-BP drug delivery system using localised administration that reduces the need for high doses of N-BP and simultaneously conveys the active ingredient to the exact location at which its effect is desired;
2. it combines the advantages of the chondroprotective and osteogenic effect of N-BP with those of an infiltration treatment based on HA (viscosupplementation, lubrication, painkilling activity, etc.);
3. it significantly increases the pharmacological efficacy of the active ingredient which, dose being equal, is much less toxic, thus avoiding the possible side effects deriving from systemic distribution or high doses;
4. it has unexpected rheological characteristics when used in the presence of bivalent cations.

The conjugate of the invention synergically exploits the properties of both N-BP and HA.

The invention is further illustrated by the following examples.

The degree of substitution is calculated by the ICP-OES (inductively coupled plasma optical emission spectrometry) method by reading the phosphorus at 213.5 nm, after digestion of the sample in nitric acid.

### Example 1. Synthesis of tetrabutylammonium alendronate salt (ALD-TBA)

The synthesis is conducted as described in the literature (Arns, S. et al., Bioorganic & Medicinal Chemistry 2012, 20: 2131-2140).

A suspension of alendronic acid (1.50 g, 6.02 mmol, 1.0 eq.) in MilliQ water (40 mL) was treated with tetrabutylammonium hydroxide (TBA-OH-30 hydrate 4.82 g, 6.03 mmol, 1.0 eq.).

The reaction mixture was stirred at room temperature for 2 hours to allow complete solubilisation, giving a clear, colourless solution. The water was removed by freeze-drying, to obtain alendronate tetrabutylammonium salt (ALD-TBA) as a hygroscopic white solid (3.1 g, 100%).

### Example 2. Synthesis of 2-chloroethyl-1H-imidazole-1-carboxylate

2-chloroethanol (3.00 mL; 0.04 mol; 1.00 eq.) was added to a solution of carbonyldiimidazole (CDI) (14.51 g; 0.09 mol; 2.00 eq.) in tetrahydrofuran (THF) (40 mL) in a flask at 0°C under constant stirring. The reaction mixture was heated to ambient temperature, and the reaction was left to proceed for 3 hours.

The solvent (THF) was removed under vacuum, and the solid product dissolved in dichloromethane and washed with 1M HCl in a separator funnel. The organic phase was dehydrated with magnesium sulphate and the solvent removed in a Rotavapor, to obtain the product as a colourless liquid (7.8 g, yield 100%).

### Example 3. Synthesis of HA-alendronate conjugate (MW of HA: 200 kDa, DS 20% mol/mol)

A solution of alendronate tetrabutylammonium salt prepared as described in Example 1 (ALD-TBA 394 mg, 0.8 mmol, 1 Eq.) in DMSO (30 mL) was treated with 2-chloroethyl-1H-imidazole-1-carboxylate (280 mg, 1.6 mmol, 2 Eq.; prepared as described in Example 2) and left to react at 40°C for 18 hours.

The reaction mixture was added to a solution of HA tetrabutylammonium salt (HA-TBA, 500 mg, 0.8 mmol, 1 Eq.) in DMSO (50 mL) and left to react at 40°C for 48 hours under stirring.

The product was purified by precipitation with the addition of a saturated solution of sodium chloride (2 mL) and ethanol (500 mL) and finally, dialysed in distilled water (Spectra/Por^{®} MWCO 20 KDa for 3 days) at pH 6 and freeze-dried. The product, obtained as sodium salt, has the appearance of a spongy white solid (380 mg, yield 95%).

### Example 4. Synthesis of HA-alendronate conjugate (MW of HA: 200 kDa, DS 10% mol/mol)

The derivative was synthesised, characterised and purified as described in Example 3, starting with 1.2 g of HA TBA salt (2 mmol, 1 Eq.) solubilised in DMSO (80 mL) and reacted with the ALD-TBA reaction mixture (1000 mg, 2 mmol, 1 Eq.) and 2-chloroethyl-1H-imidazole-1-carboxylate (470 mg, 2.45 mmol, 1.22 Eq.).

774 mg of spongy white product was obtained in the form of sodium salt. (Yield: 82%).

### Example 5. Synthesis of HA-alendronate conjugate (MW of HA: 500 kDa, DS 20% mol/mol)

The derivative was synthesised, characterised and purified as described in Example 3, starting with 1.2 g of HA TBA salt (2 mmol, 1 Eq.) solubilised in DMSO (80 mL) and reacted with the ALD-TBA reaction mixture (1000 mg, 2 mmol, 1 Eq.) and 2-chlorocthyl-1H-imidazole-1-carboxylate (470 mg, 2.45 mmol, 1.22 Eq.)

620 mg of spongy white product was obtained in the form of sodium salt. (Yield: 68%).

### Example 6. Synthesis of HA-alendronate conjugate (MW of HA: 200 kDa, DS 5% mol/mol)

The derivative was synthesised, characterised and purified as described in Example 3, starting with 1 g of HA TBA salt (1.6 mmol, 1 Eq.) solubilised in DMSO (80 mL) and reacted with the ALD-TBA reaction mixture (790 mg, 1.6 mmol, 1 Eq.) and 2-chloroethyl-1H-imidazole-1-carboxylate (140 mg, 0.8 mmol, 0.5 Eq.)

603 mg of spongy white product was obtained in the form of sodium salt. (Yield: 90%).

### Example 7. Synthesis of HA-alendronate conjugate (MW of HA: 200 kDa, DS 30% mol/mol)

The derivative was synthesised, characterised and purified as described in Example 3, starting with 1 g of HA TBA salt (1.6 mmol, 1 Eq.) solubilised in DMSO (80 mL) and reacted with the ALD-TBA reaction mixture (1.6 g, 3.2 mmol, 2 Eq.) and 2-chloroethyl-1H-imidazole-1-carboxylate (560 mg, 3.2 mmol, 0.5 Eq.)

675 mg of spongy white product was obtained in the form of sodium salt. (Yield: 85%).

### Example 8. Release of ALD from HA-alendronate conjugate. In vitro test

To demonstrate that the conjugate of the invention acts as a macromolecular DDS, the releases of ALD from the following substances were compared:
- physical mixture consisting of 200 kDa MW HA and ALD (HA+ALD);
- HA-alendronate conjugate prepared as described in Example 3 (HA-ALD);
- HA-alendronate conjugate obtained via an amido bond, prepared as described in EP 1994945 (HA-ALD amide).

All the samples contained the same dose of ALD, were in 0.1 M TRIS buffer pH 7.4, and were tested on a dialysis membrane (MWCO 10 kDa), at 37°C. The ALD release was quantified by ICP-OES analysis of phosphorus in solution, as described above.

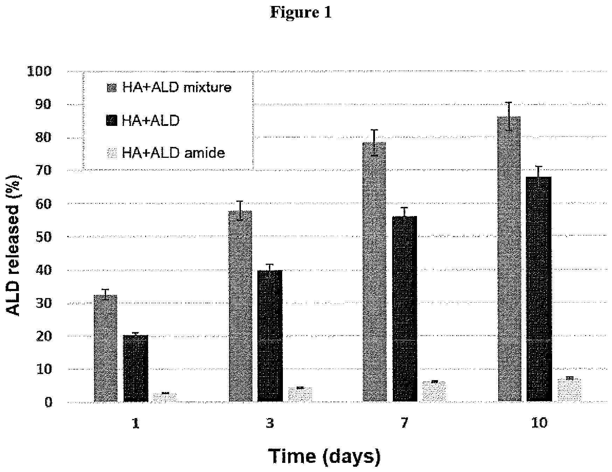

The results are set out in Figure 1.

It clearly appears that the release of ALD from the amide derivative is negligible, as expected, whereas the HA+ ALD mixture releases the active ingredient very rapidly; by the seventh day, almost 80% of the ALD has already been released, whereas the HA-alendronate conjugate of the invention has only just exceeded 50%. Its kinetics are therefore much slower, confirming that the active ingredient is released gradually following hydrolysis.

### Example 9. Rheological properties

Analysis of the viscoelastic moduli was conducted on the following samples, dissolved at the concentration specified in 0.1 M TRIS, pH=7:
- HA-alendronate conjugate prepared as described in Example 5, 20 mg/mL (containing 2.4 mg/mL of alendronate);
- 500 kDa MW HA 20 mg/mL;
- mixture of 500 kDa MW HA 20 mg/mL with alendronate (2.4 mg/mL), obtained by directly solubilising HA in an 0.1 M, pH=7 TRIS solution containing the amount of alendronate.

The samples were analysed after adding 20 µL/mL of a 100 mg/mL solution of CaCl₂ in H₂O (final CaCl₂ concentration: 2 mg/mL), to simulate as closely as possible the situation in the joint wherein, as already stated, the synovial fluid contains calcium ions even at the osteoarthrosis stage.

Analysis method: Anton Paar MCR92 rheometer; Cone 50 mm 1°; gap 0.102 mm; temperature 20°C. Frequency sweep from 0.1 to 100 rad/s, strain 10%, 15 points acquired on a logarithmic scale.

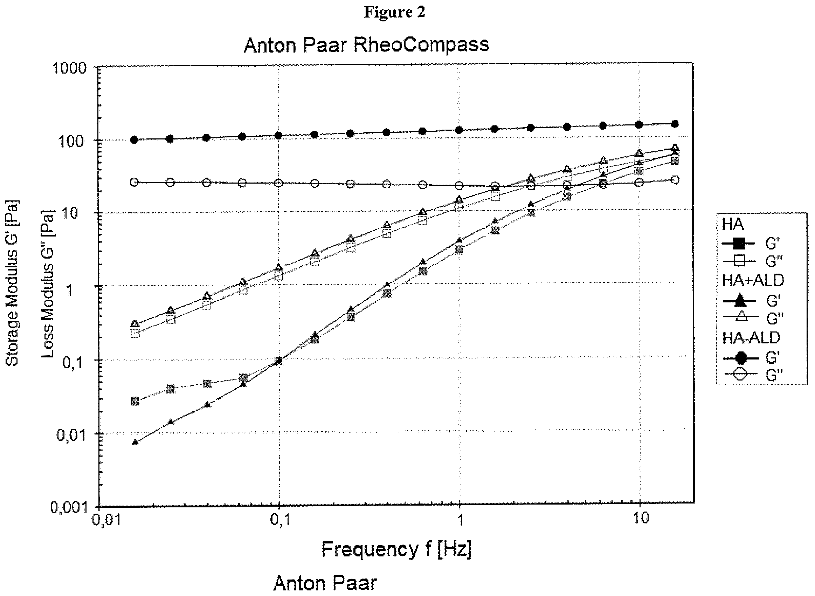

The results are described in Figure 2, which compares the values of the viscoelastic moduli; G' expresses the elastic component of the fluid, and G" the viscous component.

The moduli of the mixture and of HA alone are clearly comparable: alendronate "as is" evidently has no effect on rheological behaviour.

The behaviour of the HA-alendronate conjugate is completely different: the elastic and viscous moduli are far superior to those of the comparator species, and the values of G' and G" configure a compact gel.

By way of further confirmation of these data, the same samples were compared with two viscosupplements (Hymovis^{®} and Synvisc^{®}) among the most widely used in the intra-articular treatment of osteoarthrosis, to evaluate their dynamic viscosity (η). Dynamic viscosity, a highly significant parameter to evaluate the viscoelastic properties of a fluid, measures the shear stress of the fluid after application of a tangential force.

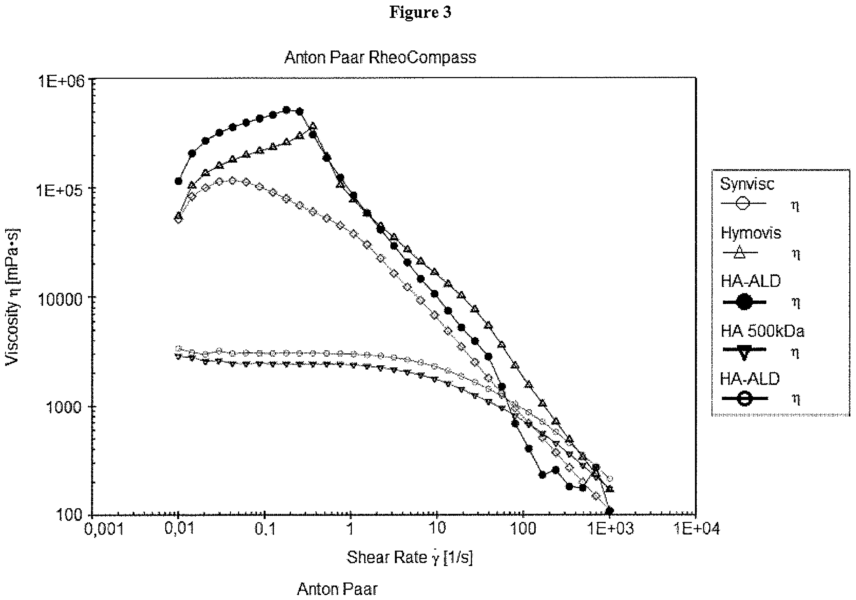

Hymovis^{®} is the hexadecyl amide of HA, which forms a compact gel due to the creation of a movable crosslink. Synvisc^{®} is a mixture of two different forms of crosslinked HA, Hylan-A and Hylan-B, in the ratio of 80:20. The crosslinking gives HA a very high MW, in the order of Millions Da. Once again the product is a compact gel. The results are set out in Figure 3.

The conjugate of the invention clearly has a high dynamic viscosity, even higher than those of the two commercial products, considered to be the first-choice products in clinical practice; however, the performance of HA alone, and of the HA+alendronate mixture, is far inferior.

The conjugate according to the invention, in terms of viscosupplementation, is therefore superior to the products currently known and used. This result is surprising as regards the dynamic viscosity values of the HA+alendronate mixture and 500kDa MW HA alone, bearing in mind that alendronate "as is" has no effect on viscosity.

This is even more significant in view of the fact that the conjugate is formulated as a viscous solution. HA-ALD releases the alendronate gradually and progressively, thus acting as a macromolecular drug delivery system, which selectively conveys the active ingredient to the site at which it is intended to act. HA-ALD can also be injected intra-articularly in the form of a viscous solution. Its administration is easy, involving small-gauge needles, and causes less pain or discomfort for the patient. Finally, HA-ALD is converted in the joint cavity to a gel with excellent rheological properties, better than those of similar products already used in the viscosupplementation field.

### Example 10. Cytotoxicity of the HA-alendronate conjugate towards osteoblasts. In vitro test

The cytotoxicity of the conjugate, associated with its ability to release alendronate in a controlled way, was evaluated by *in vitro* testing on an osteoblast cell line (Saos-2, ATCC HTB-85). The Saos-2 cells were cultured in McCoy's 5A medium (Life Technologies, cat. no. 36600-021, Italy) containing 10% foetal bovine serum (Life Technologies, cat. no. 10270106, Italy) under standard conditions (37°C, 5% CO₂) until semi-confluent. The cells were then seeded at the concentration of 1 × 10⁴ cells/well in 96-well Multiwell plates (Sarstedt, cat. no. 83,3924, Germany) and divided into three groups:
1) a control group, wherein the cells were neither treated nor stimulated;
2) a group treated with free alendronate, wherein the cells were incubated with decreasing concentrations of free alendronate for 3 and 7 days;
3) a group treated with the HA-alendronate conjugate prepared as described in Example 3, wherein the cells were treated in the same way as those in group 2.

Each sample was tested in four replications.

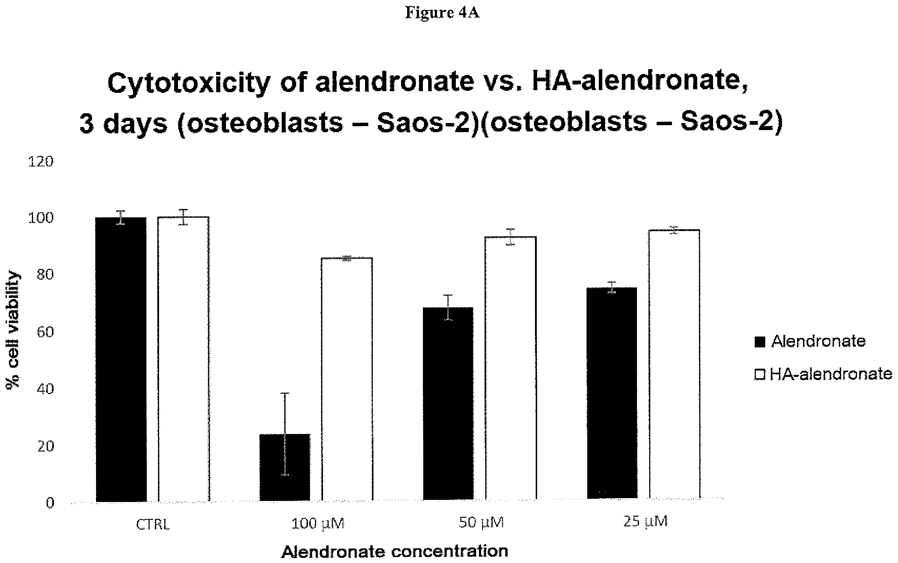

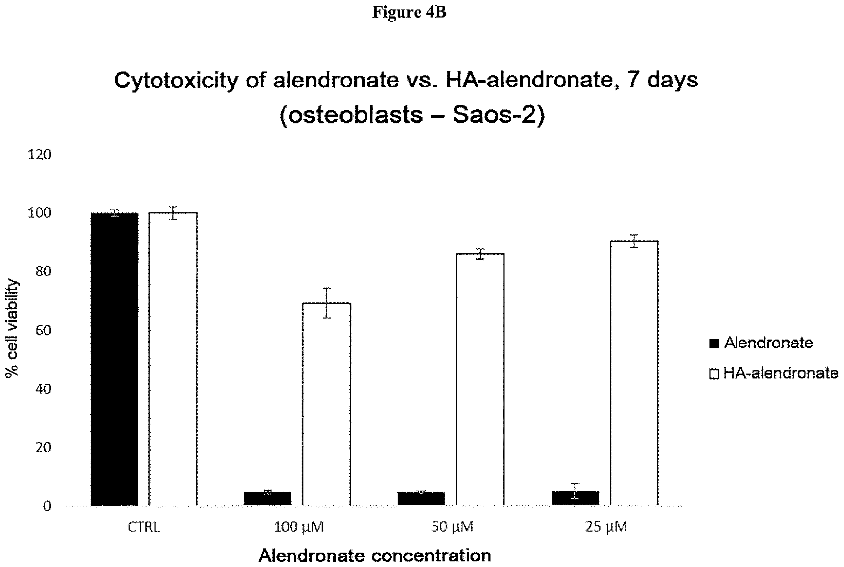

At the end of the pre-set incubation times, cell viability was quantified with the Alamar blue^{®} assay (Life Technologies, cat. no. DAL1025, Italy) according to the manufacturer's instructions, to determine cell viability on the basis of the amount of alendronate released over time. The data are set out in Figures 4A and 4B.

After 3 days of incubation (Figure 4A), a cytotoxic effect of the free alendronate was observed at concentrations of 100 µM and 50 µM (cell viability < 70% of that of the control). The cytotoxicity of free alendronate increases significantly in proportion to the incubation period.

Under the same conditions, alendronate concentration being equal, the HA-alendronate conjugate only exhibited a cytotoxic effect at the concentration of 100 µM after 7 days of incubation (Figure 4B), demonstrating that the active ingredient contained in the conjugate is released gradually. The conjugate of the invention is therefore clearly much less toxic than alendronate, dose and exposure time being equal.

### Example 11. Cytotoxicity of HA-alendronate conjugate on chondrocytes. In vitro test

The ability of the conjugate to release alendronate in a controlled way and also to have an effect on cartilage tissue was evaluated by *in vitro* testing on primary bovine chondrocytes. The primary bovine chondrocytes were isolated from the femoral condyle cartilage of an adult bovine, according to the protocol described in the literature (Mouw JK et al., Osteoarthritis and Cartilage 2005, 13: 828-836). The isolated chondrocytes were cultured in DMEM/F-12 medium (1:1) (Life Technologies, cat. no. 11320074, Italy) containing 10% foetal bovine serum (Life Technologies, cat. no. 10270106, Italy) under standard conditions (37°C, 5% CO₂), until semi-confluent. The cells were then seeded at the concentration of 1 × 10⁴ cells/well in 96-well Multiwell plates (Sarstedt, cat. no. 83.3924, Germany) and divided into three groups:
1) a control group, wherein the cells were neither treated nor stimulated;
2) a group treated with alendronate, wherein the cells were incubated with decreasing concentrations of alendronate for 3 and 7 days;
3) a group treated with the HA-alendronate conjugate prepared as described in Example 3, wherein the cells were treated in the same way as those in group 2.

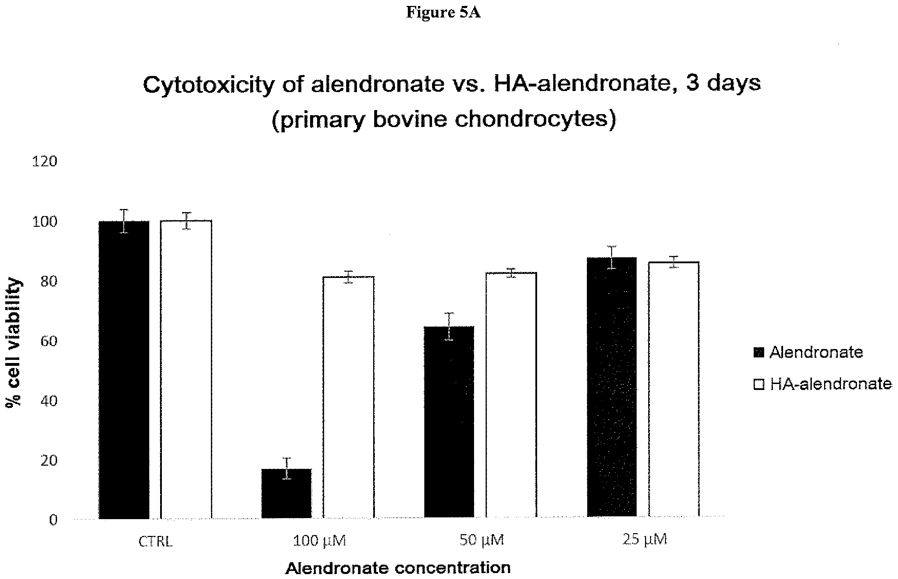

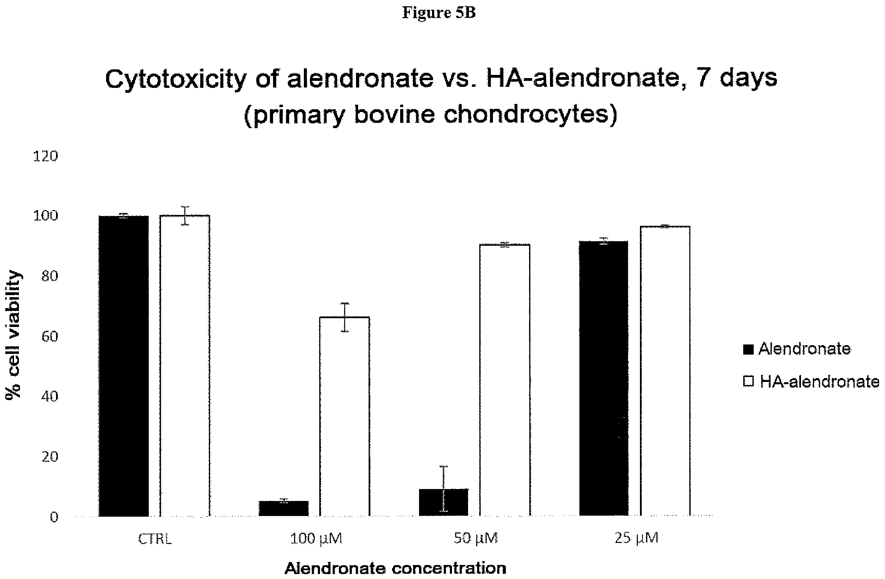

Each sample was tested in four replications. At the end of the pre-set incubation times, cell viability was quantified with the Alamar blue^{®} assay (Life Technologies, DAL1025, Italy) according to the manufacturer's instructions, to determine cell viability on the basis of the amount of alendronate released over time. The data are set out in Figures 5A and 5B.

Under the same conditions, alendronate concentration being equal, the HA-alendronate conjugate only exhibited a slight cytotoxic effect at the concentration of 100 µM after 7 days of incubation, demonstrating that the active ingredient is released gradually from the conjugate. Conversely, the free alendronate acts on the primary bovine chondrocytes after only 72 hours of incubation, and has a maximum cytotoxic effect at the concentrations of 100 µM and 50 µM after 7 days. Analysis of the data clearly demonstrates that the conjugate has considerably lower toxicity than free alendronate, especially towards osteoblasts. This aspect is particularly important since the osteoblasts are responsible for the production of organic bone matrix, and their integrity is crucial for the purpose of bone repair.

### Example 12. Release of collagen from cartilage

The efficacy of the conjugate was evaluated by measuring the amount of collagen released from a sample subjected to inflammatory stimulus, as alendronate is known to act as an anti-inflammatory. The selected model is an *ex vivo* model of cartilage inflammation after stimulus with inflammatory agents, as described in Arns, S. et al., Bioorganic & Medicinal Chemistry 2012, 20: 2131-2140. In detail, the cartilage was harvested from the patello-femoral groove and femoral condyles obtained from an adult bovine femur, and cartilage biopsies (Ø = 3 mm) were taken with a steel punch. The biopsies were cultured in a 48-well Multiwell plate (BD Falcon, cat. no. 353078, Italy) at 37°C 5% CO₂ for 24 hours in DMEM/F-12 (1:1) (Life Technologies, cat. no. 11320074, Italy) containing 2% foetal bovine serum (Life Technologies, cat. no. 10270106, Italy). After incubation, the biopsies were washed with PBS IX and divided into the following groups:
1) control group, wherein the biopsies were neither treated nor stimulated;
2) group stimulated with OSM and IL-1β pro-inflammatory cytokines (10 ng/mL each);
3) group stimulated with OSM and IL-1β and treated with HA-alendronate conjugate prepared as described in Example 3,1 mM;
4) group stimulated with OSM and IL-1β and treated with HA-alendronate conjugate prepared as described in Example 3, 0.1 mM;
5) group stimulated with OSM and IL-1β and treated with HA-alendronate conjugate prepared as described in Example 3, 0.01 mM.

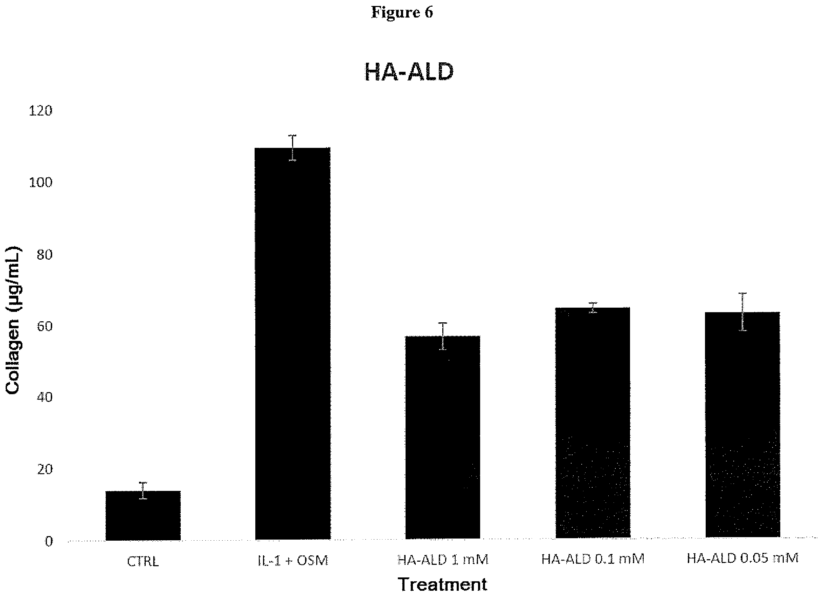

At 0, 7, 14 and 21 days, the culture medium of biopsies was aspirated and replaced with fresh culture medium containing inflammatory cytokines and HA-alendronate conjugate. After 21 days of incubation, the biopsy medium was collected, and the soluble collagen released was measured by colorimetric assay using the Sircol collagen assay kit (Biocolor, cat. no. S1000, UK) according to the manufacturer's instructions. The results are set out in Figure 6. The biopsies of the group stimulated with OSM and IL-1β release a significantly higher amount of soluble collagen into the culture medium than the control. The conjugate significantly reduces the collagen loss induced by the inflammatory stimulus, halving the amount of soluble collagen measured in the medium of the biopsies treated with a solution containing the derivative at an alendronic acid concentration of 1 mM. Said effect is also observed, to a slightly lesser extent, at the concentrations of 0.1 mM and 0.05 mM, confirming the ability of the compound to inhibit collagen release.

The proven efficacy of the conjugate of the invention in counteracting the collagen release induced by an inflammatory stimulus confirms that it maintains the pharmacological properties of alendronate, which continues to act as an anti-inflammatory, even in conjugated form.

## Claims

1. A conjugate between hyaluronic acid and an amino-bisphosphonate, of formula (I) 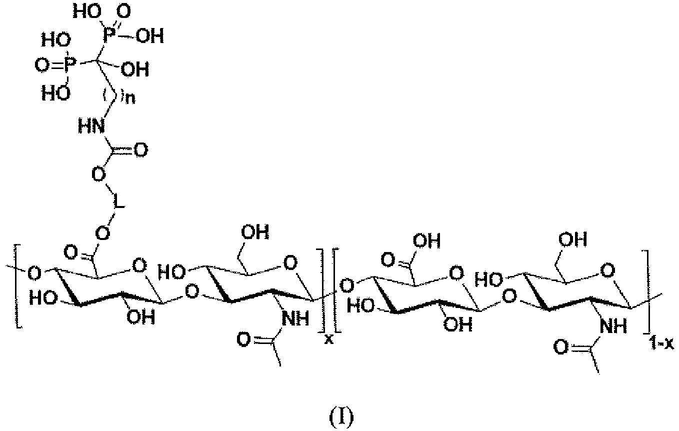 wherein:
- **L** is a spacer of formula -(CH₂)ₘ-, wherein **m** is an integer from 2 to 10; or **L** is a spacer of formula -(CH₂CH₂O)_{P}-CH₂CH₂- wherein **p** is an integer from 1 to 4;
- **n** is an integer from 2 to 5;
- **x** is a number that represents the degree of substitution (DS) of hyaluronic acid with the amino-bisphosphonate in relation to the carboxylic groups of hyaluronic acid, ranging from 0.05 to 0.30 mol of amino-bisphosphonate/mol of hyaluronic acid:
- the weight-average molecular weight of hyaluronic acid ranges from 30000 Da to 3×10⁶Da;
- the phosphonic groups of the amino-bisphosphonic moiety and the carboxyl groups of hyaluronic acid that are not involved in the conjugation with the amino-bisphosphonate are, optionally, partially or completely salified with the cation of an alkali or alkaline-earth metal, with the ammonium cation or with a (C₁-C₄)tetraalkylammonium cation, preferably with a cation of an alkaline metal, more preferably with the Na⁺ cation.

2. Conjugate according to claim 1, wherein **m** is an integer from 2 to 5.

3. Conjugate according to claim 1, wherein **p** is 1.

4. Conjugate according to any one of claims 1-3, wherein **n** is 2, 3 or 5.

5. Conjugate according to claims 1,2 and 4, wherein both **m** and **n** are 2.

6. Conjugate according to any one of the preceding claims, wherein the weight-average molecular weight of hyaluronic acid ranges from 1×10⁵ Da to 1×10⁶ Da, more preferably from 150000 Da to 800000 Da, and even more preferably from 170000 Da to 230000 Da or from 500000 Da to 730000 Da.

7. Conjugate according to any one of the preceding claims, wherein **x** ranges from 0.10 to 0.30 mol/mol, more preferably from 0.10 to 0.20 mol/mol.

8. A conjugate of formula (I) as defined in claim 1 for use as a medicament.

9. Pharmaceutical composition containing a conjugate of formula (I) as defined in claim 1, and at least one pharmaceutically acceptable excipient and/or carrier.

10. A conjugate according to claim 1 or a composition according to claim 9 for use in the intra-articular and/or locoregional treatment of osteoarthrosis and its repercussions at cartilage and subchondral level; in the treatment of post-menopausal osteoporosis or osteoporosis induced by drugs, especially steroids; in the treatment of bone fragility due to traumas or diseases, in particular multiple myeloma and bone metastases; in the intraosseous and/or locoregional treatment of diseases **characterised by** altered metabolic bone turnover, in particular Paget's disease; and in the treatment and filling of subchondral bone lesions, including bone marrow lesions.

11. A conjugate according to claim 1 or a composition according to claim 9, for use to improve the osseointegration of prostheses.

12. A viscosupplement comprising a conjugate of formula (I) as defined in claim 1, and at least one pharmaceutically acceptable excipient and/or carrier.

13. A viscosupplement according to claim 12 for use in the intra-articular and/or locoregional treatment of osteoarthrosis and its repercussions at cartilage and subchondral level; in the treatment of post-menopausal osteoporosis or osteoporosis induced by drugs, especially steroids; in the treatment of bone fragility due to traumas or diseases, in particular multiple myeloma and bone metastases; and in the intra-osseous and/or locoregional treatment of diseases **characterised by** altered metabolic bone turnover, in particular Paget's disease.

## Patentansprüche

1. Konjugat zwischen Hyaluronsäure und einem Aminobisphosphonat der Formel (I): 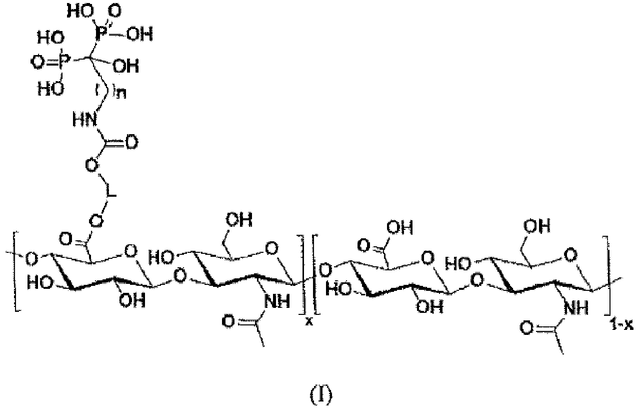 wobei:
- L ein Spacer der Formel -(CH₂)ₘ- ist, wobei m eine ganze Zahl von 2 bis 10 ist; oder L ein Spacer der Formel -(CH₂CH₂O)ₚ-CH₂CH₂- ist, wobei p eine ganze Zahl von 1 bis 4 ist;
- n eine ganze Zahl von 2 bis 5 ist;
- x eine Zahl ist, die für den Substitutionsgrad (DS) von Hyaluronsäure mit dem Aminobisphosphonat in Bezug auf die Carboxylgruppen von Hyaluronsäure steht, wobei sie im Bereich von 0,05 bis 0,30 mol Aminobisphosphonat/mol Hyaluronsäure liegt;
- das Gewichtsmittel des Molekulargewichts von Hyaluronsäure im Bereich von 30000 Da bis 3 × 10⁶ Da liegt;
- die Phosphonsäuregruppen der Aminobisphosphonat-Struktureinheit und die Carboxygruppen der Hyaluronsäure, die nicht an der Konjugation mit dem Aminobisphosphonat beteiligt sind, gegebenenfalls partiell oder vollständig mit dem Kation eines Alkali- oder Erdalkalimetalls, mit dem Ammoniumkation oder mit einem (C₁-C₄)-Tetraalkylammonium-Kation versalzt sind, vorzugsweise mit einem Kation eines Alkalimetalls, besonders bevorzugt mit dem Na⁺-Kation.

2. Konjugat gemäß Anspruch 1, wobei m eine ganze Zahl von 2 bis 5 ist.

3. Konjugat gemäß Anspruch 1, wobei p = 1 ist.

4. Konjugat gemäß einem der Ansprüche 1 bis 3, wobei n = 2, 3 oder 5 ist.

5. Konjugat gemäß Anspruch 1, 2 und 4, wobei sowohl m als auch n = 2 sind.

6. Konjugat gemäß einem der vorstehenden Ansprüche, wobei das Gewichtsmittel des Molekulargewichts von Hyaluronsäure im Bereich von 1 × 10⁵ Da bis 1 × 10⁶ Da, besonders bevorzugt 150000 Da bis 800000 Da und ganz besonders bevorzugt 170000 Da bis 230000 Da oder 500000 Da bis 730000 Da, liegt.

7. Konjugat gemäß einem der vorstehenden Ansprüche, wobei x im Bereich von 0,10 bis 0,30 mol/mol, besonders bevorzugt 0,10 bis 0,20 mol/mol, liegt.

8. Konjugat der Formel (1), wie es in Anspruch 1 definiert ist, zur Verwendung als Medikament.

9. Pharmazeutische Zusammensetzung, die ein Konjugat der Formel (1), wie es in Anspruch 1 definiert ist, und wenigstens einen pharmazeutisch annehmbaren Arzneimittelhilfsstoff und/oder Träger enthält.

10. Konjugat gemäß Anspruch 1 oder Zusammensetzung gemäß Anspruch 9 zur Verwendung bei der intraartikulären und/oder lokoregionalen Behandlung von Osteoarthrose und deren Auswirkungen auf dem Niveau des Knorpels und des Subchondriums; bei der Behandlung von postmenopausaler Osteoporose oder durch Wirkstoffe, insbesondere Steroide, induzierter Osteoporose; bei der Behandlung von Knochenbrüchigkeit aufgrund von Verletzungen oder Krankheiten, insbesondere multiplem Myelom und Knochenmetastasen; bei der intraossären und/oder lokoregionalen Behandlung von Erkrankungen, die durch einen veränderten Knochenstoffwechselumsatz gekennzeichnet sind, insbesondere Morbus Paget; und bei der Behandlung und Verfüllung von subchondrialen Knochenverletzungen einschließlich Knochenmarksläsionen.

11. Konjugat gemäß Anspruch 1 oder Zusammensetzung gemäß Anspruch 9 zur Verwendung zur Verbesserung der Osseointegration von Prothesen.

12. Viskosupplement, das ein Konjugat der Formel (I), wie es in Anspruch 1 definiert ist, und wenigstens einen pharmazeutisch annehmbaren Arzneimittelhilfsstoff und/oder Träger umfasst.

13. Viskosupplement gemäß Anspruch 12 zur Verwendung bei der intraartikulären und/oder lokoregionalen Behandlung von Osteoarthrose und deren Auswirkungen auf dem Niveau des Knorpels und des Subchondriums; bei der Behandlung von postmenopausaler Osteoporose oder durch Wirkstoffe, insbesondere Steroide, induzierter Osteoporose; bei der Behandlung von Knochenbrüchigkeit aufgrund von Verletzungen oder Krankheiten, insbesondere multiplem Myelom und Knochenmetastasen; und bei der intraossären und/oder lokoregionalen Behandlung von Erkrankungen, die durch einen veränderten Knochenstoffwechselumsatz gekennzeichnet sind, insbesondere Morbus Paget.

## Revendications

1. Conjugué entre l'acide hyaluronique et un amino-bisphosphonate, qui répond à la formule (I) : 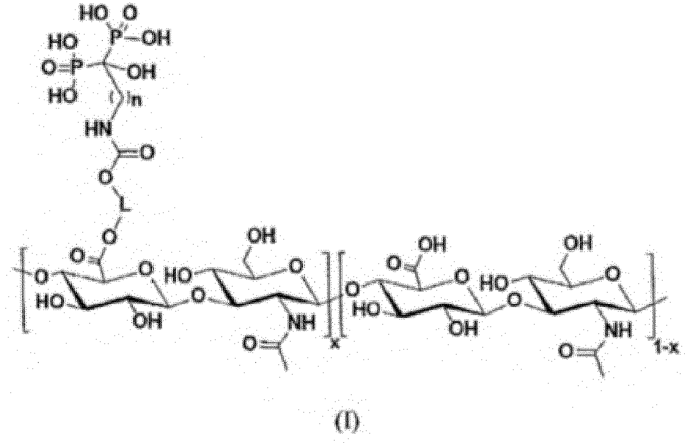 dans laquelle :
- **L** représente un espaceur répondant à la formule -(CH₂)ₘ- dans laquelle **m** représente un entier allant de 2 à 10 ; ou **L** représente un espaceur répondant à la formule -(CH₂CH₂O)_{P}-CH₂CH₂- dans laquelle **p** représente un entier allant de 1 à 4 ;
- **n** représente un entier allant de 2 à 5 ;
- **x** représente un nombre, à savoir le degré de substitution (DS) de l'acide hyaluronique avec l'amino-bisphosphonate par rapport aux groupes carboxyliques de l'acide hyaluronique, qui se situe dans la plage allant de 0,05 à 0,30 mole de l'amino-bisphosphonate/mole de l'acide hyaluronique ;
- le poids moléculaire moyen en poids de l'acide hyaluronique se situe dans la plage allant de 30.000 Da à 3 × 10⁶ Da ;
- les groupes phosphoniques de la fraction amino-bisphosphonique et les groupes carboxyle de l'acide hyaluronique qui ne sont pas impliqués dans la conjugaison avec l'amino-bisphosphonate sont, de manière facultative, salifiés en partie ou totalement avec le cation d'un métal alcalin ou d'un métal alcalino-terreux, avec le cation d'ammonium ou avec le cation de tétraalkyl(en C₁-C₄)ammonium, de préférence avec un cation d'un métal alcalin, de manière plus préférée avec le cation de Na⁺.

2. Conjugué selon la revendication 1, dans lequel **m** représente un entier allant de 2 à 5.

3. Conjugué selon la revendication 1, dans lequel **p** est égal à 1.

4. Conjugué selon l'une quelconque des revendications 1 à 3, dans lequel **n** est égal à 2, 3 ou 5.

5. Conjugué selon les revendications 1, 2 et 4, dans lequel **m** et **n** sont tous deux égaux à 2.

6. Conjugué selon l'une quelconque des revendications précédentes, dans lequel le poids moléculaire moyen en poids de l'acide hyaluronique se situe dans une plage allant de 1 × 10⁵ Da à 1 × 10⁶ Da, de manière plus préférée de 150.000 Da à 800.000 Da, et de manière encore plus préférée de 170.000 Da à 230.000 Da ou de 500.000 Da à 730.000 Da.

7. Conjugué selon l'une quelconque des revendications précédentes, dans lequel **x** se situe dans une plage allant de 0,10 à 0,30 mole/mole, de manière plus préférée allant de 0,10 à 0,20 mole/mole.

8. Conjugué répondant à la formule (I) telle qu'elle a été définie à la revendication 1, pour son utilisation comme médicament.

9. Composition pharmaceutique qui contient un conjugué répondant à la formule (I) telle qu'elle a été définie à la revendication 1 et au moins un excipient et/ou un support pharmaceutiquement acceptable(s).

10. Conjugué selon la revendication 1 ou composition selon la revendication 9 pour son utilisation dans le traitement intra-articulaire et/ou loco-régional de l'ostéoarthrite et de ses répercussions au niveau du cartilage et au niveau sous-chondral ; dans le traitement de l'ostéoporose post-ménopausique ou de l'ostéoporose induite par des médicaments, en particulier des stéroïdes ; dans le traitement d'une fragilité osseuse qui est due à des traumatismes ou à des maladies, en particulier le myélome multiple et des métastases osseuses ; dans le traitement intra-osseux et/ou loco-régional de maladies **caractérisées par** un renouvellement altéré du métabolisme osseux, en particulier la maladie de Paget ; et dans le traitement et le remplissage de lésions osseuses sous-chondrales, y compris des lésions de la moelle osseuse.

11. Conjugué selon la revendication 1 ou composition selon la revendication 9 pour son utilisation dans l'amélioration de l'ostéointégration de prothèses.

12. Viscosupplément qui comprend un conjugué répondant à la formule (I) telle qu'elle a été définie à la revendication 1 et au moins un excipient et/ou un support pharmaceutiquement acceptable(s).

13. Viscosupplément selon la revendication 12, pour son utilisation dans le traitement intra-articulaire et/ou loco-régional de l'ostéoarthrite et de ses répercussions au niveau du cartilage et au niveau sous-chondral ; dans le traitement de l'ostéoporose post-ménopausique ou de l'ostéoporose induite par des médicaments, en particulier des stéroïdes ; dans le traitement d'une fragilité osseuse qui est due à des traumatismes ou à des maladies, en particulier le myélome multiple et des métastases osseuses ; et dans le traitement intra-osseux et/ou loco-régional de maladies **caractérisées par** un renouvellement altéré du métabolisme osseux, en particulier la maladie de Paget.
